(19) Europäisches Patentamt
European Patent Office
Office européen des brevets

(11) Veröffentlichungsnummer: **0 396 069 B1**

(12) **EUROPÄISCHE PATENTSCHRIFT**

(45) Veröffentlichungstag der Patentschrift: **22.06.94**

(51) Int. Cl.⁵: **C07D 403/04**, A61K 31/55, A61K 31/50, //(C07D403/04, 237:00,223:00)

(21) Anmeldenummer: **90108172.9**

(22) Anmeldetag: **28.04.90**

(54) **Salze des Azelastins mit verbesserter Löslichkeit.**

(30) Priorität: **05.05.89 DE 3914859**

(43) Veröffentlichungstag der Anmeldung:
**07.11.90 Patentblatt 90/45**

(45) Bekanntmachung des Hinweises auf die Patenterteilung:
**22.06.94 Patentblatt 94/25**

(84) Benannte Vertragsstaaten:
**AT BE CH DE DK ES FR GB GR IT LI LU NL SE**

(56) Entgegenhaltungen:
**EP-A- 0 174 464**
**EP-A- 0 289 939**
**EP-A- 0 316 639**
**DE-A- 2 164 058**

(73) Patentinhaber: **ASTA Medica Aktiengesellschaft**
**An der Pikardie 10**
**D-01277 Dresden(DE)**

(72) Erfinder: **Hettche, Helmut, Dr.**
**Martinstrasse 23**
**D-6057 Dietzenbach(DE)**
Erfinder: **Muckenschnabel, Reinhard, Dr.**
**c/o Laboratoires Sarget S.A.,**
**Avenue du President**
**J.F.Kennedy, B.P.100, F-33701 Merignac,(FR)**
Erfinder: **Scheffler, Gerhard, Dr.**
**Lindenallee 54**
**D-6454 Bruchköbel(DE)**
Erfinder: **Ilona, Fleischhauer, Dr.**
**Zum Mühler 9**
**D-6050 Offenbach(DE)**
Erfinder: **Morick, Wolfgang, Dr.**
**Vogelweidstrasse 7**
**D-6000 Frankturt 70(DE)**

EP 0 396 069 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Azelastin ist ein Phthalazinon-Derivat folgender Strukturformel :

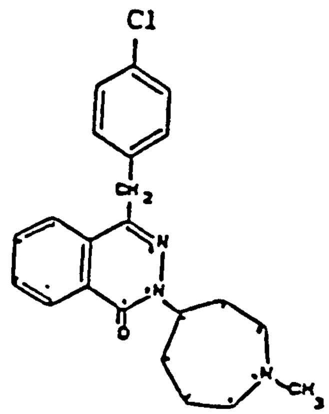

Die chemische Bezeichnung ist: 4-(4-Chlorbenzyl)-2-(perhydro-1-methylazepin-4-yl)-1-(2H) phthalazinon. Azelastin wird insbesondere zur Asthmaprophylaxe eingesetzt. Azelastin hat ebenfalls antiallergische und antihistaminische Eigenschaften, siehe DE-PS 21 64 058.

Azelastin löst sich in Wasser bei 20 ° C zu 0,005 %.

Bisher angewendete Salze von Azelastin weisen in Wasser bei 20 ° C folgende Löslichkeit auf:

| | |
|---|---|
| Hydrochlorid | 1 % |
| Embonat | 0,0015 % |

Weitere Salze des Azelastins haben folgende Löslichkeiten:

| | |
|---|---|
| Succinat<br>Phosphat<br>Maleat<br>Citrat<br>Methansulfonat | Löslichkeit in Wasser 20° ca. 1 % |
| Fumarat<br>Toluolsulfonat<br>Benzolsulfonat<br>Tartrat | unter 1 % |

Die angegebenen Löslichkeiten sowie die ausgeprägte Abhängigkeit der Löslichkeit von der Temperatur führen dazu, daß die Salze zur Herstellung von pharmazeutischen Zubereitungen mit einem höheren Gehalt an gelöstem Azelastin nicht geeignet sind. Insbesondere die Herstellung von lokal zu applizierenden Zubereitungen, insbesondere derjenigen mit einem begrenzten Gehalt an Wasser (zum Beispiel Emulsions-Salben und Cremes sowie Gele) erfordert die Verfügbarkeit höher konzentrierter wässriger Lösungen, um die erforderlichen Wirkstoffmengen auf die Haut oder Schleimhaut aufzubringen.

Es ist daher Aufgabe der Erfindung, Salze des Azelastins mit einer höheren Löslichkeit in Wasser bereitzustellen und die Löslichkeit dieser Salze zusätzlich zu steigern, auf der anderen Seite aber die Verträglichkeit der damit hergestellten Zubereitungen zum Beispiel auf der Haut oder Schleimhaut nicht negativ zu beeinflussen.

Es wurde überraschend gefunden, daß sich aus Salzen des Azelastins mit Essigsäure, Gluconsäure, Milchsäure oder Äpfelsäure (Azelastinacetat, Azelastingluconat, Azelastinlaktat oder Azelastinmalat) stabile pharmazeutische Zubereitungen, insbesondere Lösungen, herstellen lassen, die einen hohen Gehalt an Wirkstoff aufweisen (beispielsweise bis zu 50 %).
Das zur Herstellung dieser Salze verwendete Azelastin ist in der DE-PS 21 64 058 beschrieben.

Die Herstellung der genannten Salze erfolgt durch Umsetzung von 4-(4-Chlorbenzyl)-2(perhydro-1-methyl-azepin-4-yl)-1-(2H) phthalazinon mit Essigsäure, Gluconsäure oder Gluconsäure-δ-lacton, Milchsäure oder Äpfelsäure. Diese Umsetzung kann mit oder ohne Lösungsmittel bei Temperaturen zwischen 20 °C und 140 °C, vorzugsweise 50 ° und 120 °C erfolgen. Vorzugsweise werden die Komponenten jeweils in molarem Verhältnis umgesetzt (1 Mol Azelastin mit 1 Mol Säure). Ein geringer Überschuß (zum Beispiel bis 0,1 Mol) an Säure ist möglich. Falls die Salze nicht isoliert werden, das heißt falls diese in Form von Lösungen oder Suspensionen verwendet werden, kann die Säure in größerem Überschuß verwendet werden, und zwar bis maximal 1 Mol Überschuß (also insgesamt 2 Mol Säure pro 1 Mol Azelastin) vorzugsweise bis 0,5 Mol Überschuß.
Als Lösungsmittel für diese Umsetzung kommen beispielsweise niedere Alkohole (zum Beispiel mit 1-6 C-Atomen wie Methanol, Ethanol, n-Propanol, Isopropanol, n-Butanol, 1,2-Propandiol, 1,4-Butandiol, n-Pentanol, 3-Pentanol, n-Hexanol), niedere Ketone (zum Beispiel mit 1-6 C-Atomen wie Aceton, 2-Butanon, Methylisobutylketon), Polyalkylenglykole (wobei alkylen 2,3 oder 4 C-Atome bedeutet) mit Molekulargewichten zwischen 190 und 7500 und cyclische gesättigte Alkohole mit 4 bis 8, vorzugsweise 4 bis 6 C-Atomen wie zum Beispiel Glycofurol sowie pharmazeutisch verwendbare organische Säuren und Mischungen dieser Mittel mit Wasser in Frage.

Falls man anstelle von Gluconsäure das Gluconsäure-δ-lacton einsetzt, muß eine gewisse Menge Wasser vorhanden sein (zum Beispiel 1 Mol Wasser pro 1 Mol Lacton). Es ist beispielsweise vorteilhaft, das Gluconsäure-δ-lacton einzusetzen, da es handelsüblich in kristalliner Form vorliegt und schnell in Wasser zu Gluconsäure hydrolysiert wird. Beispielsweise kann die Herstellung auf folgende Weise erfolgen:

Azelastin wird gegebenenfalls in einem Lösungsmittel (Ethanol, 1,2-Propandiol) suspendiert und mit einer bei einer Temperatur zwischen 20 °C und 140 °C bereiteten Lösung von 1 Mol Gluconsäure-δ-lacton in einem wasserhaltigen Lösungsmittel (Ethanol, 1,2-Propandiol) oder mit 1 Mol der genannten Säuren mit oder ohne Lösungsmittel versetzt. Das Reaktionsgemisch läßt man noch einige Zeit, vorzugsweise unter Erwärmen (50 bis 70 °C), nachreagieren und verdampft dann gegebenenfalls vorhandene Lösungsmittel. Der verbleibende Rückstand wird mit Alkohol nachgedampft und bei erhöhter Temperatur im Vakuum getrocknet.
Das Salz wird in einer Ausbeute von 90 % gewonnen.

Die erfindungsgemäßen Salze in fester Form haben folgende Schmelzpunkte:

| | | | |
|---|---|---|---|
| Acetat: | 116-118 °C | Gluconat: | 109,2 - 109,8 °C |
| Laktat: | 139-140 °C | Malat: | 152,5 - 153,1 °C |

Acetat, Lactat und Malat des Azelastins werden in Form weißer kristalliner Pulver erhalten. Das Gluconat wird als gelbliches kristallines Pulver erhalten.

Die Löslichkeiten der erfindungsgemäßen Salze des Azelastins beträgt zum Beispiel in Wasser bei Raumtemperatur:

| | |
|---|---|
| Acetat: | mehr als 10 % |
| Gluconat: | bis zu 55 % |
| Lactat: | mehr als 10 % |
| Malat: | 3 % |

Lösungen von Azelastinacetat, -gluconat, -laktat oder -malat lassen sich auch dadurch erhalten, daß man Azelastin in Form der Base mit der entsprechenden Säure in Wasser oder wässrigen Lösungsmitteln (Polyethylenglykol-Wasser, Propylenglykol-Wasser) vermischt (zum Beispiel durch Rühren oder Suspendieren bei einer Temperatur zwischen 20 °C und 80 °C).

Falls hierbei anstelle von Gluconsäure das Gluconsäure-δ-lacton eingesetzt wird, ist die Anwesenheit von Wasser erforderlich, und zwar mindestens 1 Mol Wasser, bezogen auf 1 Mol Gluconsäure-δ-lacton.

Durch Zusatz überschüssiger Mengen an Säure wird die Löslichkeit der Salze gesteigert. Beispielsweise wählt man einen 1-fachen, vorzugsweise 0,5-fachen, insbesondere 0,25-fachen Überschuß an Säure.

Auch durch den Zusatz eines Alkalisalzes (Na, K) oder Ammoniumsalzes der betreffenden Säure (zum Beispiel bis 1 Mol Alkali- oder Ammoniumsalz, insbesondere bis 0,5 Mol Salz pro 1 Mol Azelastinsalz) kann die Löslichkeit zusätzlich gesteigert werden.
Der pH-Wert der Lösungen beziehungsweise Zubereitungen unter Verwendung der erfindungsgemäßen Salze liegt im sauren Bereich im allgemeinen nicht unter 3, vorzugsweise nicht unter 3,5, insbesondere nicht unter 4, im oberen Bereich im allgemeinen nicht über 8, vorzugsweise nicht über 7,5.

Beispielsweise wird durch Zusatz der Säuren folgende Löslichkeit und folgender pH-Wert der wässrigen Lösung erhalten:

| Salz | Überschuß Säure (Mol) | Löslichkeit | pH-Wert |
|---|---|---|---|
| Acetat | 0<br>0,1<br>0,25 | 2 %<br>3 %<br>10 % | 6,35<br>5,52<br>5,1 |
| Gluconat | 0,5 | 10 % | 4,5 |
| Laktat | 0<br>0,1<br>0,25 | 3 %<br>5 %<br>10 % | 5,15<br>4,6<br>4,1 |
| Malat | 0 | 5 % | 3,85 |

Die Lösungen verbleiben auch bei 7tägiger Aufbewahrungszeit bei 25 °C und Tageslicht sowie nach 7tägigem Stehen im Kühlschrank bei 7 °C ohne Kristallbildung beziehungsweise Trübung klar gelöst. Die erfindungsgemäß hergestellten Lösungen weisen demnach Vorteile in der Stabilität und in der verbesserten Lagerhaltung auf.
Solche Lösungen sind wässrige Lösungen, die zum Beispiel auch übliche Stabilisatoren, Puffersubstanzen sowie sonstige übliche physiologisch verwendbare Hilfsstoffe enthalten können. Es handelt sich entweder um rein wässrige Lösungen oder um wässrige Lösungen, wo ein Teil des Wassers durch ein oder mehrere andere physiologisch verträgliche flüssige Mittel (bis zu 20 Gewichts%) ersetzt ist. Als solche flüssigen Mittel kommen zum Beispiel vorzugsweise in Frage: $C_2$-$C_4$-Alkanole, flüssige Polyethylenglykole (zum Beispiel bis Molgewicht 1500).

Die Herstellung der pharmazeutischen Darreichungsformen aus den Azelastinsalzen oder deren Lösungen erfolgt nach üblichen Standardverfahren.

So können Lösungen, Tinkturen, Lotionen, Emulsionen, Gele, Cremes, Salben, Shampoos und Pflaster hergestellt werden.

Die Konzentrationen an Azelastin (bezogen auf die Base) können zum Beispiel zwischen 0,1 und 50 Gewichts% liegen, vorzugsweise zwischen 1 und 20 Gewichts%, insbesondere 2 bis 10 Gewichts% oder auch zwischen 3 und 5 Gewichts%.
Hohe Konzentrationen in Lösungen (insbesondere wässrigen Lösungen) zwischen 20 und 50 Gewichts% kommen insbesondere für das Azelastin-Gluconat in Betracht. Selbstverständlich kann das Gluconat auch in niedrigeren Konzentrationen als 20 Gewichts% verwendet werden. Konzentrationen in Lösungen bis zu 20 Gewichts% kommen insbesondere für das Acetat und Lactat in Frage. Konzentrationen in Lösungen bis zu 5 % kommen insbesondere für das Malat in Frage.
Beispielsweise kommen für die einzelnen Salze in den Lösungen (vorzugsweise wässrigen Lösungen) insbesondere folgende Konzentrationen in Frage

| | |
|---|---|
| Azelastin-Gluconat: | 0,1-50, vorzugsweise 1-50 Gewichts% |
| Azelastin-Acetat: | 0,1-20, vorzugsweise 1-20 Gewichts% |
| Azelastin-Lactat: | 0,1-20, vorzugsweise 1-20 Gewichts% |
| Azelastin-Malat: | 0,1- 5, vorzugsweise 1- 3 Gewichts% |

Der Vorzug dieser Arzneiformen liegt einerseits in der verbesserten Stabilität. So kommt es gegenüber den aus bisher bekannten Salzen hergestellten Arzneiformen nicht zu Kristallabscheidungen und Emulsionstrennungen. Der andere Vorzug ist deren verbesserte Wirksamkeit, die durch die höhere Konzentration und auch die erhöhte Permeation des Azelastins durch Haut und Schleimhaut bedingt ist.

Die pharmazeutischen Zusammensetzungen beziehungsweise Arzneimittel enthalten als Wirkstoff Azelastin oder seine physiologisch verträglichen Salze. Der Wirkstoff liegt gegebenenfalls in Mischung mit anderen pharmakologisch beziehungsweise pharmazeutisch wirksamen Stoffen vor. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind: Ullmanns Encyclopädie der technischen Chemie, Band 4 (1953), Seite 1 bis 39; Journal of Pharmaceutical Sciences, Band 52 (1963) Seite 918 und ff., H.v.Czetsch-Lindenwald, Hilfsstoffe für Pharmazie und angrenzende Gebiete; Pharm. Ind., Heft 2, 1961, Seite 72 und ff.; Dr. H.P. Fiedler, Lexikon der Hilfsstoffe für Pharmazie, Kosmetik und angrenzende Gebiete Cantor KG. Aulendorf in Württemberg 1981.

Beispiele hierfür sind Gelatine, natürliche Zucker wie Rohrzucker oder Milchzucker, Lecithin, Pektin, Stärke (zum Beispiel Maisstärke) , Cyclodextrine und Cyclodextrinderivate, Polyvinylpyrrolidon, Polyvinylacetat, Gelatine, Gummi arabicum, Alginsäure, Tylose, Talkum, Lycopodium, Kieselsäure (zum Beispiel kolloidale), Cellulose, Cellulosederivate (zum Beispiel Celluloseether, bei denen die Cellulose-Hydroxygruppen teilweise mit niederen gesättigten aliphatischen Alkoholen und/oder niederen gesättigten aliphatischen Oxyalkoholen verethert sind, zum Beispiel Methyloxypropylcellulose, Methylcellulose, Hydroxypropylmethylcellulose, Hydroxypropylmethylcellulosephthalat); Fettsäuren sowie Magnesium-, Calcium- oder Aluminiumsalze von Fettsäuren mit 12 bis 22 C-Atomen, insbesondere der gesättigten (zum Beispiel Stearate), Emulgatoren, Öle und Fette, insbesondere pflanzliche (zum Beispiel Erdnußöl , Rizinusöl, Olivenöl, Sesamöl Baumwollsaatöl, Maisöl, Weizenkeimöl, Sonnenblumensamenöl, Kabeljau-Leberöl, jeweils auch hydriert; Mono-, Di - und Triglyceride aus gesättigten Fettsäuren $C_{12}H_{24}O_2$ bis $C_{18}H_{36}O_2$ und deren Gemische), pharmazeutisch verträgliche ein- oder mehrwertige Alkohole und Polyglykole wie Polyethylenglykole sowie Derivate hiervon, Ester von aliphatischen gesättigten oder ungesättigten Fettsäuren (2 bis 22 C-Atome, insbesondere 10 - 18 C-Atome) mit einwertigen aliphatischen Alkoholen (1 bis 20 C-Atome) oder mehrwertigen Alkoholen wie Glykolen, Glycerin, Diethylenglykol Pentaerythrit, Sorbit, Mannit und so weiter, die gegebenenfalls auch verethert sein können, Ester der Zitronensäure mit primären Alkoholen, Essigsäure, Benzylbenzoat, Dioxolane, Glyzerinformale, Tetrahydrofurfurylalkohol, Polyglykolether mit $C_1$-$C_{12}$-Alkoholen, Dimethylacetamid, Lactamide, Lactate, Ethylcarbonate, Silicone (insbesondere mittelviskose Polydimethylsiloxane).

Zur Herstellung von Lösungen kommen beispielsweise Wasser oder physiologisch verträgliche organische Lösungsmittel in Frage, wie zum Beispiel Ethanol, 1,2-Propylenglykol, Polyglykole und deren Derivate, Dimethylsulfoxid, Fettalkohole, Triglyceride, Partialester des Glycerins, Paraffine und ähnliche.

Für injizierbare Lösungen oder Suspensionen kommen zum Beispiel nicht-toxische parenteral verträgliche Verdunnungsmittel oder Lösungsmittel in Frage, wie zum Beispiel : Wasser, 1,3-Butandiol, Ethanol, 1,2-Propylenglykol, Polyglykole in Mischung mit Wasser, Ringer's Lösung, isotonische Kochsalzlösung oder auch gehärtete Öle einschließlich synthetischer Mono- oder Diglyceride oder Fettsäuren wie Oleinsäure.

Bei der Herstellung der Zubereitungen können bekannte und übliche Lösungsvermittler, beziehungsweise Emulgatoren, verwendet werden. Als Lösungsvermittler und Emulgatoren kommen beispielsweise in Frage: Polyvinylpyrrolidon, Sorbitanfettsäureester wie Sorbitantrioleat, Phosphatide, wie Lecithin, Acacia, Traganth, polyoxyethyliertes Sorbitanmonooleat und andere ethoxylierte Fettsäureester des Sorbitan, polyoxyethylierte Fette, polyoxyethylierte Oleotriglyceride, linolisierte Oleotriglyceride, Polyethylenoxyd-Kondensationsprodukte von Fettalkoholen, Alkylphenolen oder Fettsäuren oder auch 1-Methyl-3-(2-hydroxyethyl)-imidazolidon-(2). Polyoxyethyliert bedeutet hierbei, daß die betreffenden Stoffe Polyoxyethyl enketten enthalten, deren Polymerisationsgrad im allgemeinen zwischen 2 bis 40 und insbesondere zwischen 10 bis 20 liegt.

Solche polyoxyethylierten Stoffe können beispielsweise durch Umsetzung von hydroxylgruppenhaltigen Verbindungen (beispielsweise Mono- oder Diglyceride oder ungesättigte Verbindungen wie zum Beispiel solchen, die Ölsäurereste enthalten) mit Ethylenoxyd erhalten werden (zum Beispiel 40 Mol Ethylenoxyd pro Mol Glycerid).

Beispiele für Oleotriglyceride sind Olivenöl, Erdnußöl, Rizinusöl, Sesamöl, Baumwollsaatöl, Maisöl.

Siehe auch Dr. H. P. Fiedler "Lexikon der Hilfsstoffe" für Pharmazie, Kosmetik und angrenzende Gebiete" 1971, S. 191-195.

Darüberhinaus ist der Zusatz von Konservierungsmitteln, Stabilisatoren, Puffersubstanzen, zum Beispiel Calciumhydrogenphosphat, kolloidales Aluminiumhydroxyd, Farbstoffen, Antioxydantien und Komplexbildnern (zum Beispiel Ethylendiaminotetraessigsäure) und dergleichen möglich.

Gegebenenfalls ist zur Stabilisierung des Wirkstoffmoleküls mit physiologisch verträglichen Säuren oder

Puffern auf einen pH-Bereich von ca. 3 bis 7 einzustellen. Im allgemeinen wird ein möglichst neutraler bis schwach saurer (bis pH 3,5) pH-Wert bevorzugt.

Zur Herstellung von dermal anzuwendenden Zubereitungen kommen in Frage die vorgenannten Substanzen und streichfähige oder flüssige Kohlenwasserstoffe wie Vaselin oder Paraffin oder Gele aus Paraffinkohlenwasserstoffen und Polyethylen, Fette und Öle pflanzlichen oder tierischen Ursprungs, die zum Teil auch hydriert sein können oder synthetische Fette wie Glyceride der Fettsäuren $C_8$-$C_{18}$, sodann Bienenwachs, Cetylpalmitat, Wollwachs, Wollwachsalkohole; Fettalkohole wie Cetylalkohol, Stearylalkohol, Polyethylenglykole vom Molekulargewicht 200 bis 20 000; flüssige Wachse wie Isopropylmyristat, Isopropylstearat, Äthyloleat; Emulgatoren wie Natrium-, Kalium-, Ammoniumsalze der Stearinsäure oder Palmitinsäure sowie Triethanolaminstearat, Alkalisalze der Ölsäure, Ricinolsäure, Salze von sulfurierten Fettalkoholen wie Natrium-laurylsulfat, Natriumcetylsulfat, Natriumstearylsulfat, Salze der Gallensäure, Sterole wie Cholesterol, Partialfettsäureester mehrwertiger Alkohole wie Ethylenglykolmonostearat, Glycerolmonostearat, Pentaerythritmonostearat, Partialfettsäureester des Sorbitans, Partialfettsäureester des Polyoxyethylensorbitans, Sorbitolether des Polyoxyethylens, Fettsäureester des Polyoxyethylens, Fettalkoholether des Polyoxyethylens, Fettsäureester der Saccarose, Fettsäureester des Polyglycerols, Lecithin.

Als Antioxydantien kommen beispielsweise Natriummetabisulfit, Ascorbinsäure, Gallussäure, Gallussäurealkylester, Butylhydroxyanisol, Nordihydroguajaretsäure, Tocopherole sowie Tocopherole + Synergisten (Stoffe, die Schwermetalle durch Komplexbildung binden, beispielsweise Lecithin, Ascorbinsäure, Phosphorsäure) zur Anwendung. Der Zusatz der Synergisten steigert die antioxygene Wirkung der Tocopherole erheblich.

Als Konservierungsmittel kommen beispielsweise Sorbinsäure, p-Hydroxybenzoesäureester (zum Beispiel Niederalkylester), Benzoesäure, Natriumbenzoat, Trichlorisobutylalkohol, Phenol, Kresol, Benzethoniumchlorid, und Formalinderivate in Betracht.

Die pharmazeutische und galenische Handhabung der Wirkstoffe erfolgt nach den üblichen Standardmethoden. Beispielsweise werden Wirkstoff (e) und Hilfs- beziehungsweise Trägerstoffe durch Rühren oder Homogenisieren (zum Beispiel mittels üblicher Mischgeräte) gut vermischt, wobei im allgemeinen bei Temperaturen zwischen 20 und 80° C, vorzugsweise 20 bis 50° C, insbesondere bei Raumtemperatur gearbeitet wird. Im übrigen wird auf das folgende Standardwerk verwiesen: Sucker, Fuchs, Speiser, Pharmazeutische Technologie, Thieme-Verlag Stuttgart, 1978.

Die Applikation kann auf die Haut oder Schleimhaut oder in das Körperinnere erfolgen, beispielsweise oral, enteral, pulmonal, rectal, nasal, vaginal lingual, intravenös, intraarteriell, intrakardial intramuskulär, intraperitoneal , intracutan, subcutan.

Bei den parenteralen Zubereitungsformen handelt es sich insbesondere um sterile beziehungsweise sterilisierte Erzeugnisse.

Falls die verwendeten Säuren optisch aktive Kohlenstoffatome enthalten, können die erfindungsgemäßen Salze in Form der Racemate, der eventuellen diastereomeren Formen sowie in Form der optisch aktiven rechts-und linksdrehenden Formen vorliegen. Herstellung erfolgt durch Verwendung der entsprechendden Racemate oder optisch aktiven Formen der Ausgangssäuren, oder durch anschließende Racematspaltung beziehungsweise Trennung der erfindungsgemäßen Salze nach den hierfür üblichen Methoden.

Die IR-Spektren des Acetats, Malats, Lactats und Gluconats werden durch Figur 1, Figur 2, Figur 3 und Figur 4 wiedergegeben.

Die pharmakologische Wirkung der erfindungsgemäßen Azelastin-Salze entspricht derjenigen des Azelastins (zum Beispiel derjenigen des Azelastin-hydrochlorids). Sie sind also antiallergisch (histaminolytisch) und asthmaprophylaktisch wirksam. Darüberhinaus weisen sie ebenso wie das Azelastin entzündungshemmende und cytoprotektive Wirkungen auf und sind wirksam gegen Psoriasis-Erkrankungen.
Von den Entzündungserkrankungen kommen insbesondere auch Colitis ulcerosa und verwandte Erkrankungen (zum Beispiel Morbus Crohn, entzündliche Darmerkrankungen (inflammatory bowel desease), Gastritis, Weichteilrheumatismus, entzündliche und degenerative Formen des Rheumatismus) in Frage.
Unter Psoriasis-Erkrankungen werden verstanden: Hauterkrankungen, die mit Hyperkeratosen einhergehen, insbesondere Psoriasis.

Zur histaminolytischen beziehungsweise antiallergischen Wirkung: Die erfindungsgemäßen Salze zeichnen sich durch eine außerordentlich hohe Aktivität bei parenteraler und vor allem bei oraler Applikation sowie durch eine lange Wirkungsdauer aus (zum Beispiel im Histamin-Aerosol-Versuch am Meerschweinchen oder im Histamin- beziehungsweise Histamin-Liberator-Quaddeltest am Menschen).
Am Meerschweinchen wurde die histaminolytische (antiallergische) Wirkung im Histamin-Aerosol-Versuch getestet. Die Tiere atmeten ein Aerosol einer wäßrigen Lösung von Histamindihydrochlorid (Konzentration 4 mg/ml). Die Inhalation führte bei unbehandelten Tieren innerhalb von 2 Minuten zu schwerster Dyspnoe

(Erstickungskrämpfe, Seitenlage).
Die asthmaprophylaktische Wirkung kann zum Beispiel im Screening-Modell "Ovalbumin-Asthma" an wachen Meerschweinchen 2 Stunden vor Allergenexposition bei per-oraler Gabe bestimmt werden. Die $ED_{50}$ bei diesem Versuch für die erfindungsgemäßen Salze liegt im Durchschnitt bei circa 0,3 mg/kg.

Zur Feststellung der histaminolytischen Wirkung wurden die Substanzen Gruppen von 8-10 Tieren subcutan oder oral appliziert. Anschließend wurden die Tiere zu verschiedenen Zeiten der Einwirkung des Histamin-Aerosols ausgesetzt. Sie galten als geschützt, wenn sie ohne schwere Dyspnoe (Seitenlage) 10 Minuten lang die Inhalation des Aerosols tolerierten. Zur Auswertung wurden mit Hilfe der Probit-Analyse aus der Beziehung zwischen den Dosen-Logarithmen und den Schutzhäufigkeiten die mitteleren wirksamen Dosen (DE 50 {mg/kg}) bestimmt.

Die Wirkung geht aus folgenden Tabellen hervor:

| Histaminolytische Wirkung im Histamin-Aerosol-Versuch am Meerschweinchen, Application subcutan 1 Stunde (1 h) vor dem Aerosol | |
|---|---|
| | DE 50 (mg/kg) |
| erfindungsgemäße Verbindungen | 0,031 |

| Histaminolytische Wirkung im Histamin-Aerosol-Versuch am Meerschweinchen, Application per os 2 Stunden (2 h) und 8 Stunden (8 h) vor dem Aerosol | | |
|---|---|---|
| | DE 50 (mg/kg) | |
| | 2 h-Wert | 8 h-Wert |
| erfindungsgemäße Verbindungen | 0,037 | 0,029 |

Zur entzündungshemmenden und cytoprotektiven Wirkung:

Beispielsweise wird beim Arachidonsäure-induzierten Mäuseohrödem bei einer Dosis von 3 mg peroral/kg Körpergewicht Maus eine 20 - 30%ige Hemmung des Ödems und bei topischer Applikation bei einer Dosis von 0,25 mg/Mäuseohr eine 30 - 40%ige Hemmung erzielt. Am Rattenpfotenödem (induziert durch Carrageenin, Bestimmung der Pfotenvolumina nach einer Stunde) wird zum Beispiel bei einer peroralen Dosis von 3,5 - 4,5 mg/kg Ratte eine 50%ige Hemmung der Ödembildung erzielt. Die niedrigste, bereits wirksame Dosis in den oben angegebenen Tierversuchen ist beispielsweise 1 - 2 mg/kg oral beziehungsweise 10 mg/kg bei topischer Anwendung (10 mg/kg entsprechen etwa 0,25 $mg/cm^2$ Körperoberfläche).
Die erfindungsgemäßen Verbindungen hemmen ebenfalls die durch Indomethacin ausgelöste ulzeröse intestinale Entzündung an der Ratte (P. Del Soldato et al., Agents and Actions, Band 16, 5, Birkhäuser Verlag, Basel 1985, Seiten 393-396) in einem Dosisbereich von beispielsweise 8-80 mg/kg dosisabhängig. Dieses Modell zeigt insbesondere die Wirkung bei Colitis ulcerosa an. Beispielsweise wird bei oben genannter Versuchsmethode bei 5maliger Verabreichung von jeweils 50 mg/kg Körpergewicht Ratte peroral (Applikations-Schema nach Del Soldato) eine rund 50%ige Hemmung der Entzundung erzielt.

Zur Wirkung gegen Psoriasis-Erkrankungen und Prüfung dieser Wirkung:

Mäuse (Durchschnittsgewicht 25 g) erhielten 7 Tage lang täglich 1 mg/kg erfindungsgemäßes Salz oral. Am 8. Tag wurde die obere Hautschicht mit Sandpapier entfernt.

Durch diese mechanische Irritation und Entfernung der obersten Hautschicht entsteht eine akute Reaktion, die morphologische Ähnlichkeiten zur psoriatischen Reaktion aufweist. Außerdem erhöht sich der Leukotrien-Gehalt in der Dermis und Epidermis. Dies geht mit einer Erniedrigung der Prostaglandin-Konzentration einher. Auch diese Veränderungen sind for Psoriasis typisch. Durch die Vorbehandlung mit den erfindungsgemäßen Salzen fanden diese Veränderungen nicht statt. Diese normalisieren beziehungsweise erniedrigen sogar die dermale beziehungsweise epidermale Leukotrien-Konzentration und erhöhen die Konzentration der Prostaglandine.

Beispiel 1

Lösung mit 3,47 % Azelastinacetat (entsprechend 3 % Azelastin)

86,24 ml 1N-Essigsäure werden unter Rühren mit 850 ml gereinigtem Wasser gemischt. Zu der Mischung werden 30 g Azelastin gegeben und so lange gerührt, bis eine klare Lösung entstanden ist. Anschließend wird mit gereinigtem Wasser auf 1000 ml aufgefüllt.

Der pH-Wert der erhaltenen Lösung beträgt 5,5, das molare Verhältnis Azelastin : Essigsäure beträgt 1 : 1,1.

Beispiel 2

Creme mit 5,785 % Azelastinacetat (entsprechend 5 % Azelastin)

50 g Polyoxyethylen-40-stearat (Handelsname: Myrj® 52), 80 g Cetylstearylalkohol, 200 g weißes Vaselin, 150 g dickflüssiges Paraffin und 5 g Polydimethylsiloxan (Dimethicone) werden in einer Homogenisierapparatur bei 80 °C zusammengeschmolzen. In der Schmelze werden 1,26 g Methyl-4-hydroxybenzoat und 0,533 g Propyl-4-hydroxybenzoat gelöst.

298,207 g gereinigtes Wasser werden auf 70°C erhitzt und darin 1,4 g Methyl-4-hydroxybenzoat und 0,6 g Propyl-4-hydroxybenzoat gelöst. Dieser Lösung werden 163 g 1N-Essigsäure zugefügt. In der erhaltenen Lösung werden 50 g Azelastin unter Rühren gelöst. Die etwa 70 °C warme Lösung wird langsam und unter Rühren der oben erhaltenen, auf etwa 80 °C temperierten Fettschmelze zugefügt. Die entstandene Emulsion wird homogenisiert und unter Rühren auf Raumtemperatur abgekühlt.

Der pH-Wert der Emulsion beträgt 5,2, das molare Verhältnis Azelastin : Essigsäure beträgt 1 : 1,25.

Beispiel 3

Creme mit 3,71 % Azelastinlaktat (entsprechend 3 % Azelastin)

470,877 g gereinigtes Wasser werden auf etwa 70 °C erwärmt und darin 1,4 g Methyl-4-hydroxybenzoat und 0,6 g Propyl-4-hydroxybenzoat gelöst. Dieser Lösung werden 10,33 g 90%ige Milchsäure zugegeben und in der Lösung 30 g Azelastin unter Rühren aufgelöst.

Die etwa 70 °C warme Lösung wird langsam und unter Rohren der auf etwa 80 °C temperierten Fettschmelze aus Beispiel 2 zugefügt. Die entstandene Emulsion wird homogenisiert und unter Rühren auf Raumtemperatur abgekühlt.

Der pH-Wert der Emulsion beträgt 4,5, das molare Verhältnis Azelastin : Milchsäure beträgt 1 : 1,1.

Beispiel 4

Gel mit 15,13 % Azelastingluconat (entsprechend 10 % Azelastin)

796,3 g gereinigtes Wasser werden auf etwa 70 °C erhitzt und darin 1 g Methyl-4-hydroxybenzoat und 0,4 g Propyl-4-hydroxybenzoat gelöst. In der Lösung werden anschließend 58,3 g Gluconsäure-delta-lacton gelöst.

Die Lösung wird 1 Stunde bei einer Temperatur von 70 °C gehalten, wobei Gluconsäure-delta-lacton zu Gluconsäure hydrolysiert. Anschließend werden in der Lösung 100 g Azelastin unter Ruhren aufgelöst.

Der auf Raumtemperatur abgekühlten Lösung werden nacheinander 20 g Polyoxyethylen(20 Mol Ethylenoxid)sorbitan-laurat (Polysorbat 20) und 24 g Hydroxyethylcellulose (molarer Substitutionsgrad : 2,5, Viskosität der 2%igen Lösung : 100 000 mPa.sec) zugefügt.

Anschließend wird so lange gerührt, bis ein klares Gel entstanden ist.

pH-Wert des Gels : 4,0

Das molare Verhältnis Azelastin : Gluconsäure beträgt 1 : 1,25.

Beispiel 5

Fettsalbe mit 4,05 % Azelastinmalat (entsprechend 3 % Azelastin)

779,5 g weißes Vaselin und 30 g Polyoxyethylen-20-stearylether (Handelsname Brij® 78) werden bei einer Temperatur von etwa 75 °C zusammengeschmolzen. 40,5 g Azelastinmalat werden in 150 g dickflüssigem Paraffin suspendiert. Dieser Suspension wird die oben erhaltene Schmelze unter Rühren zugegeben. Anschließend wird die Salbe unter Rühren auf Raumtemperatur abgekühlt.

## Patentansprüche

**1.** Salze des Azelastins mit Essigsäure, Gluconsäure, Milchsäure oder Äpfelsäure.

**2.** Verfahren zur Herstellung von Salzen gemäß Anspruch 1, dadurch gekennzeichnet, daß Azelastin mit oder ohne Lösungsmitteln bei Temperaturen zwischen 20 und 140 °C mit Essigsäure, Gluconsäure, Gluconsäure-δ-lacton, Milchsäure oder Apfelsäure umgesetzt wird.

**3.** Arzneimittel , die Salze gemäß Anspruch 1 gegebenenfalls mit weiteren üblichen Hilfs- und Trägerstoffen beziehungsweise Verdünnungsmitteln enthalten.

**4.** Arzneimittel nach Anspruch 3, dadurch gekennzeichnet, daß der Azelastingehalt bezogen auf die Azelastinbase zwischen 0,1 bis 50 Gewichts%, vorzugsweise 2 bis 10 Gewichts%, insbesondere 3 bis 5 Gewichts% liegt.

**5.** Arzneimittel gemäß Anspruch 3, dadurch gekennzeichnet, daß es sich um Lösungen der erfindungsgemäßen Salze nach Anspruch 1 handelt, wobei die Azelastinkonzentration bezogen auf die Base in solchen Lösungen zwischen 0,1 und 50 Gewichts%, vorzugsweise zwischen 2 und 10 Gewichts%, insbesondere 3 bis 5 Gewichts% liegt.

**6.** Verfahren zur Herstellung von Arzneimitteln, dadurch gekennzeichnet, daß mindestens ein Salz gemäß Anspruch 1 mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht wird.

**7.** Verfahren zur Herstellung eines Arzneimittels, dadurch gekennzeichnet, daß man mindestens ein Salz gemäß Anspruch 1 zusammen mit üblichen Träger- und/oder Verdünnungs- beziehungsweise Hilfsstoffen bei Temperaturen zwischen 0 und 120 °C, vorzugsweise 20 bis 80 °C, vermischt beziehungsweise homogenisiert und gegebenenfalls die so erhaltene Mischung zur Herstellung von Zubereitungen, die in der Dosierungseinheit 0,1 mg bis 1 g, vorzugsweise 1 bis 100 mg Wirkstoff gemäß Anspruch 1 (bezogen auf die Base) enthalten, in Hohlzellen entsprechender Größe ausgießt, zu Tabletten verpreßt oder in Kapseln entsprechender Größe abfüllt und granuliert und dann gegebenenfalls unter Zusatz von weiteren üblichen Hilfsstoffen zu Tabletten verpreßt oder in Kapseln abfüllt.

**8.** Verfahren zur Herstellung eines Arzneimittels, dadurch gekennzeichnet, daß man mindestens ein Salz gemäß Anspruch 1 mit einem oder mehreren der folgenden Stoffe: Stärke, Cyclodextrin, Harnstoff, Cellulose, Lactose, Formalin-Casein, modifizierte Stärke, Magnesiumstearat, Calciumhydrogenphosphat, Kieselsäure, Talkum vermischt, die erhaltene Mischung gegebenenfalls mit einer wässrigen Lösung, die als Bestandteil mindestens Gelatine, Stärke, Polyvinylpyrrolidon, Vinylpyrrolidon-Vinylacetat-Copolymerisat und/oder Polyoxyethylensorbitanmonooleat enthält, granuliert, das Granulat gegebenenfalls mit einem oder mehreren der oben genannten Hilfsstoffe homogenisiert, und die Mischung zu Tabletten verpreßt oder in Kapseln abfüllt, wobei die Tabletten oder Kapseln in der Dosierungseinheit jeweils 0,1 mg bis 100 mg, vorzugsweise 1 bis 20 mg Wirkstoff gemäß Anspruch 1 (bezogen auf die Base) enthalten.

**9.** Verfahren zur Herstellung eines Arzneimittels, dadurch gekennzeichnet, daß man mindestens ein Salz gemäß Anspruch 1 bei Temperaturen zwischen 31 und 65 °C in geschmolzenem Hartfett oder anderen, Fettsäureglyceride enthaltenden Mischungen mischt oder suspendiert und homogenisiert, und anschließend die Mischung in Hohlzellen ausgießt oder in Kapseln abfüllt, wobei die Dosierungseinheit 0,1 mg

bis 100 g, vorzugsweise 0,5 bis 20 mg Wirkstoff gemäß Anspruch 1 (bezogen auf die Base) enthält.

**10.** Verfahren zur Herstellung eines Arzneimittels, dadurch gekennzeichnet, daß man mindestens ein Salz gemäß Anspruch 1 bei einer Temperatur zwischen 20 bis 120 ° C, gegebenenfalls in Gegenwart eines und/oder mehrerer Emulgatoren und/oder Komplexbildnern mit mindestens einem der folgenden Stoffe zu einer Mischung, die 0,1 bis 50 Gewichts% Wirkstoff gemäß Anspruch 1 (bezogen auf die Base) homogenisiert und/oder emulgiert: Wasser, Glycerin, Paraffin, Vaseline, aliphatischer Alkohol mit 12 bis 25 C-Atomen, aliphatische Monocarbonsäure mit 15 bis 20 C-Atomen, Sorbitanmonopalmitat, Polyoxyethylenpolyolfettsäureester, ein- oder mehrwertiger niedrigmolekularer aliphatischer Alkohol, Fettsäureglycerid, Wachs, Silikon, Polyethylenglykol

**11.** Verfahren zur Herstellung eines Arzneimittels, dadurch gekennzeichnet, daß man ein Salz gemäß Anspruch 1 bei Temperaturen zwischen 20 und 100 ° C, gegebenenfalls in Anwesenheit eines Emulgators, in Wasser, physiologisch unbedenklichen Alkoholen, Dimethylsulfoxid oder Polyethylenglykol oder Mischungen hiervon auflöst und gegebenenfalls die so erhaltene Lösung mit soviel Wasser, Alkohol Dimethylsulfoxid oder Polyethylenglykol auffüllt, daß die Endlösung 1 bis 50 Gewichts% an Wirkstoff gemäß Anspruch 1 (bezogen auf die Base) enthält.

**12.** Verwendung von Salzen gemäß Anspruch 1 zur Herstellung von Arzneimitteln.

## Claims

**1.** Salts of azelastine with acetic acid, gluconic acid, lactic acid or malic acid.

**2.** A process for the production of the salts claimed in claim 1, characterized in that azelastine is reacted with acetic acid, gluconic acid, gluconic acid δ-lactone, lactic acid or malic acid in the presence or absence of solvents at temperatures in the range from 20 to 140 ° C.

**3.** Medicaments containing the salts claimed in claim 1, optionally together with other typical auxiliaries and carriers or diluents.

**4.** Medicaments as claimed in claim 3, characterized in that the azelastine content, based on the azelastine base, is between 0.1 and 50% by weight, preferably between 2 and 10% by weight and more preferably between 3 and 5% by weight.

**5.** Medicaments as claimed in claim 3, characterized in that they are solutions of the salts according to the invention as claimed in claim 1 with azelastine concentrations, based on the base, of 0.1 to 50% by weight, preferably 2 to 10% by weight and more preferably 3 to 5% by weight.

**6.** A process for the production of medicaments, characterized in that at least one salt according to claim 1 is processed to pharmaceutical preparations or brought into a therapeutically useable form with typical pharmaceutical carriers and/or diluents or other auxiliaries.

**7.** A process for the production of a medicament, characterized in that at least one salt according to claim 1 is mixed or homogenized together with typical carriers and/or diluents or auxiliaries at temperatures of 0 to 120 ° C and preferably at temperatures of 20 to 80 ° C and, to produce preparations containing 0.1 mg to 1 g and preferably 1 to 100 mg of active substance according to claim 1 (based on the base) per dosage unit, the mixture thus obtained is optionally poured into hollow cells of corresponding size, pressed to tablets or introduced into capsules of corresponding size and is granulated and then pressed to tablets, optionally together with other typical auxiliaries, or introduced into capsules.

**8.** A process for the production of a medicament, characterized in that at least one salt according to claim 1 is mixed with one or more of the following substances: starch, cyclodextrin, urea, cellulose, lactose, formalin-casein, modified starch, magnesium stearate, calcium hydrogen phosphate, silica, talcum, the mixture obtained is granulated, optionally with an aqueous solution containing at least gelatine, starch, polyvinyl pyrrolidone, vinyl pyrrolidone/vinyl acetate copolymer and/or polyoxyethylene sorbitan monooleate as a constituent, the granules obtained are optionally homogenized with one or more of the auxiliaries mentioned above and the resulting mixture is pressed to tablets or introduced into capsules,

the tablets or capsules containing 0.1 mg to 100 mg and preferably 1 to 20 mg of the active substance according to claim 1 (based on the base) per dosage unit.

**9.** A process for the production of a medicament, characterized in that at least one salt according to claim 1 is mixed or suspended and homogenized in molten hard fat or other mixtures containing fatty acid glycerides at temperatures of 31 to 65 °C and the mixture obtained is poured out into hollow cells or introduced into capsules, each dosage unit containing 0.1 mg to 100 g and preferably 0.5 to 20 mg active substance according to claim 1 (based on the base).

**10.** A process for the production of a medicament, characterized in that at least one salt according to claim 1 is homogenized and/or emulsified with at least one of the following substances to form a mixture containing 0.1 to 50% by weight of active substance according to claim 1 (based on the base): water, glycerol, paraffin, vaseline, $C_{12-25}$ aliphatic alcohol, $C_{15-20}$ aliphatic monocarboxylic acid, sorbitan monopalmitate, polyoxyethylene polyol fatty acid ester, monohydric or polyhydric low molecular weight aliphatic alcohol, fatty acid glyceride, wax, silicone, polyethylene glycol, at a temperature in the range from 20 to 120 °C, optionally in the presence of one and/or more emulsifiers and/or complexing agents.

**11.** A process for the production of a medicament, characterized in that a salt according to claim 1 is dissolved in water, physiologically safe alcohols, dimethyl sulfoxide or polyethylene glycol or mixtures thereof, optionally in the presence of an emulsifier, at temperatures in the range from 20 to 100 °C and the solution obtained is optionally made up with water, alcohol, dimethyl sulfoxide or polyethylene glycol in such a quantity that the final solution contains 1 to 50% by weight of active substance according to claim 1 (based on the base).

**12.** The use of the salts claimed in claim 1 for the production of medicaments.

**Revendications**

**1.** Sels de l'azélastine avec de l'acide acétique, de l'acide gluconique, de l'acide lactique ou de l'acide malique.

**2.** Procédé de préparation de sels selon la revendication 1, caractérisé en ce que de l'azélastine est mise en réaction, avec ou sans solvants, à des températures comprises entre 20 et 140 °C, avec de l'acide acétique, de l'acide gluconique, de la δ-gluconolactone, de l'acide lactique ou de l'acide malique.

**3.** Médicaments qui contiennent des sels selon la revendication 1, éventuellement avec d'autres adjuvants et excipients ou diluants usuels.

**4.** Médicaments selon la revendication 3, caractérisés en ce que leur teneur en azélastine, ramenée a l'azélastine-base, se situe entre 0,1 et 50% en poids, de préférence entre 2 et 10% en poids, en particulier entre 3 et 5% en poids.

**5.** Médicaments selon la revendication 3, caractérisés en ce qu'il s'agit de solutions des sels faisant l'objet de l'invention selon la revendication 1, la concentration d'azélastine rapportée à la base, dans de telles solutions, se situant entre 0,1 et 50% en poids, de préférence entre 2 et 10% en poids, en particulier entre 3 et 5% en poids.

**6.** Procédé de préparation de médicaments, caractérisé en ce qu'on travaille au moins un sel selon la revendication 1 avec des excipients et/ou des diluants ou d'autres adjuvants pharmaceutiques usuels pour former des préparations pharmaceutiques, ou on le met sous une forme thérapeutiquement utilisable .

**7.** Procédé de préparation d'un médicament, caractérisé en ce qu'au moins un sel selon la revendication 1 est mélangé ou homogénéisé avec des excipients et/ou des diluants ou des adjuvants usuels, à des températures comprises entre 0 et 120 °C, de préférence entre 20 et 80 °C, et éventuellement le mélange ainsi obtenu est coulé dans des cavités de taille appropriée, pressé en comprimés ou chargé dans des capsules de taille appropriée, ou bien il est granulé, puis pressé en comprimés, éventuellement avec addition d'autres adjuvants usuels, ou chargé dans des capsules, pour la fabrication de

préparations qui contiennent, par unité posologique, de 0,1 mg à 1 g, de préférence de 1 à 100 mg de substance active selon la revendication 1 (rapportés à la base).

**8.** Procédé de préparation d'un médicament, caractérisé en ce qu'au moins un sel selon la revendication 1 est mélangé avec une au moins des substances suivantes; amidon, cyclodextrine, urée, cellulose, lactose, formaline-caséine, amidon modifié, stéarate de magnésium, hydrogénophosphate de calcium, acide silicique, talc, le mélange obtenu est éventuellement granulé avec une solution aqueuse qui contient, comme constituant, au moins de la gélatine, de l'amidon, de la polyvinylpyrrolidone, un copolymère vinylpyrrolidone/acétate de vinyle et/ou du monooléate de polyoxyéthylènesorbitanne, le granulé est homogénéisé éventuellement avec un ou plusieurs des adjuvants susmentionnés, et le mélange est pressé en comprimés ou chargé dans des capsules, les comprimés ou les capsules contenant chacun, par unité posologique, de 0,1 mg à 100 mg, de préférence de 1 à 20 mg de substance active selon la revendication 1 (rapportés à la base).

**9.** Procédé de préparation d'un médicament, caractérisé en ce qu'on mélange ou on met en suspension et on homogénéise au moins un sel selon la revendication 1, à des températures comprises entre 31 et 65 °C, dans de la graisse dure fondue ou dans d'autres mélanges contenant des glycérides d'acides gras, puis on coule le mélange dans des cavités ou on le charge dans des capsules, l'unité posologique contenant de 0,1 mg à 100 g, de préférence de 0,5 à 20 mg de substance active selon la revendication 1 (rapportés à la base).

**10.** Procédé de préparation d'un médicament, caractérisé en ce qu'on homognénéise et/ou on émulsifie au moins un sel selon la revendication 1, à une température comprise entre 20 et 120 °C, éventuellement en présence d'un et/ou de plusieurs émulsifiants et/ou complexants, avec au moins l'une des substances suivantes: eau, glycérine, paraffine, vaseline, alcool aliphatique à 12-25 atomes de carbone, acide monocarboxylique aliphatique à 15-20 atomes de carbone, monopalmitate de sorbitanne, ester de polyoxyéthylènepolyol d'acide gras, mono- ou polyalcool aliphatique de bas poids moléculaire, glycéride d'acide gras, cire, silicone, polyéthylèneglycol, pour former un mélange qui contient de 0,1 à 50% en poids de substance active selon la revendication 1 (rapportés à la base).

**11.** Procédé de préparation d'un médicament, caractérisé en ce qu'on dissout un sel selon la revendication 1, à des températures comprises entre 20 et 100 °C, éventuellement en présence d'un émulsifiant, dans de l'eau, dans des alcools acceptables physiologiquement, dans du diméthylsulfoxyde, dans du poyéthylèneglycol ou dans des mélanges de ces substances, et on complète éventuellement la solution ainsi obtenue avec la quantité nécessaire d'eau, d'alcool, de diméthylsulfoxyde ou de polyéthylèneglycol pour que la solution finale contienne de 1 à 50% en poids de substance active selon la revendication 1 (rapportés à la base).

**12.** Utilisation de sels selon la revendication 1 pour la préparation de médicaments.

Fig. 1

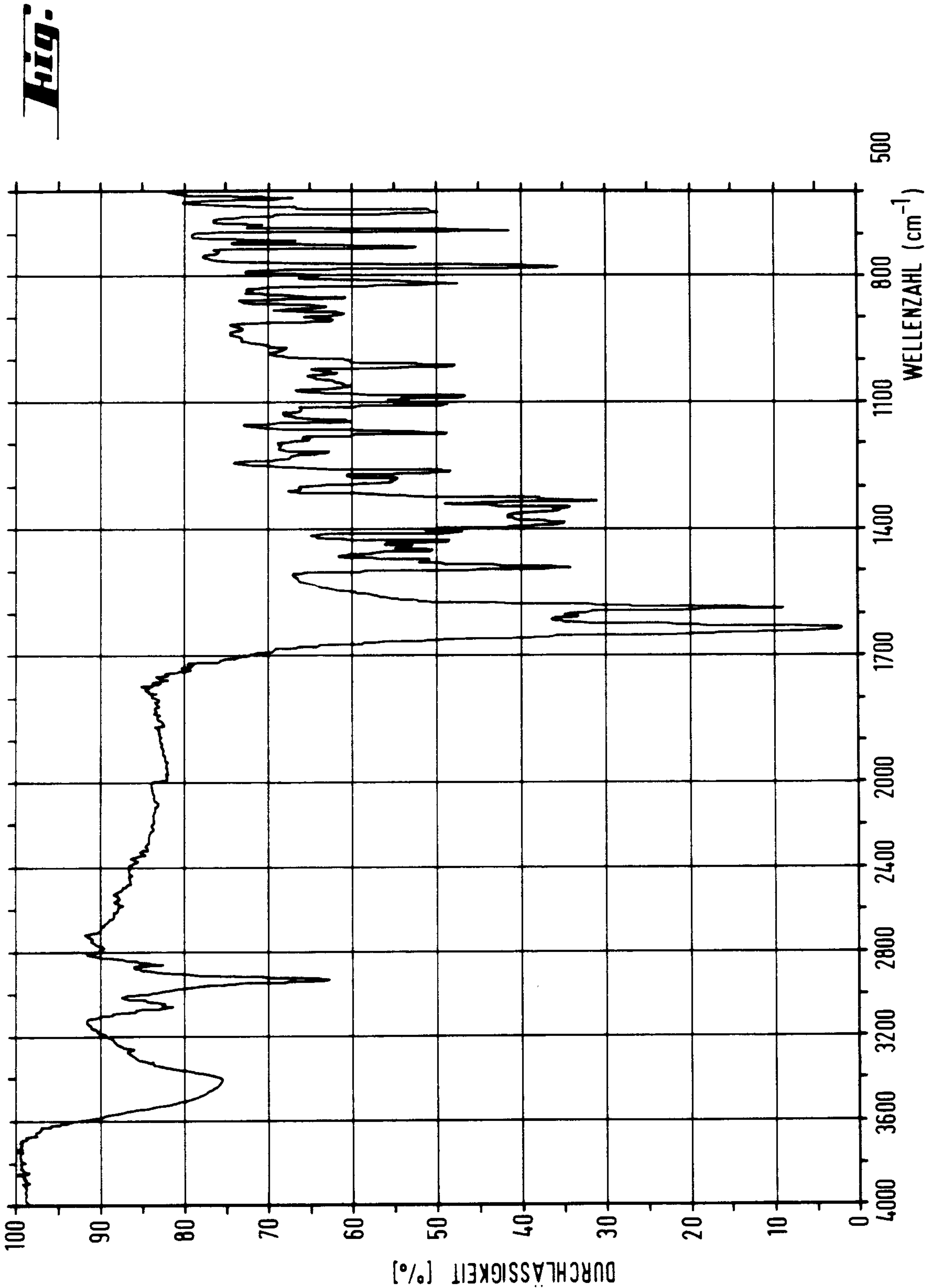
DURCHLÄSSIGKEIT [%]
100
90
80
70
60
50
40
30
20
10
0
WELLENZAHL (cm-1)
4000
3600
3200
2800
2400
2000
1700
1400
1100
800
500

Fig. 2

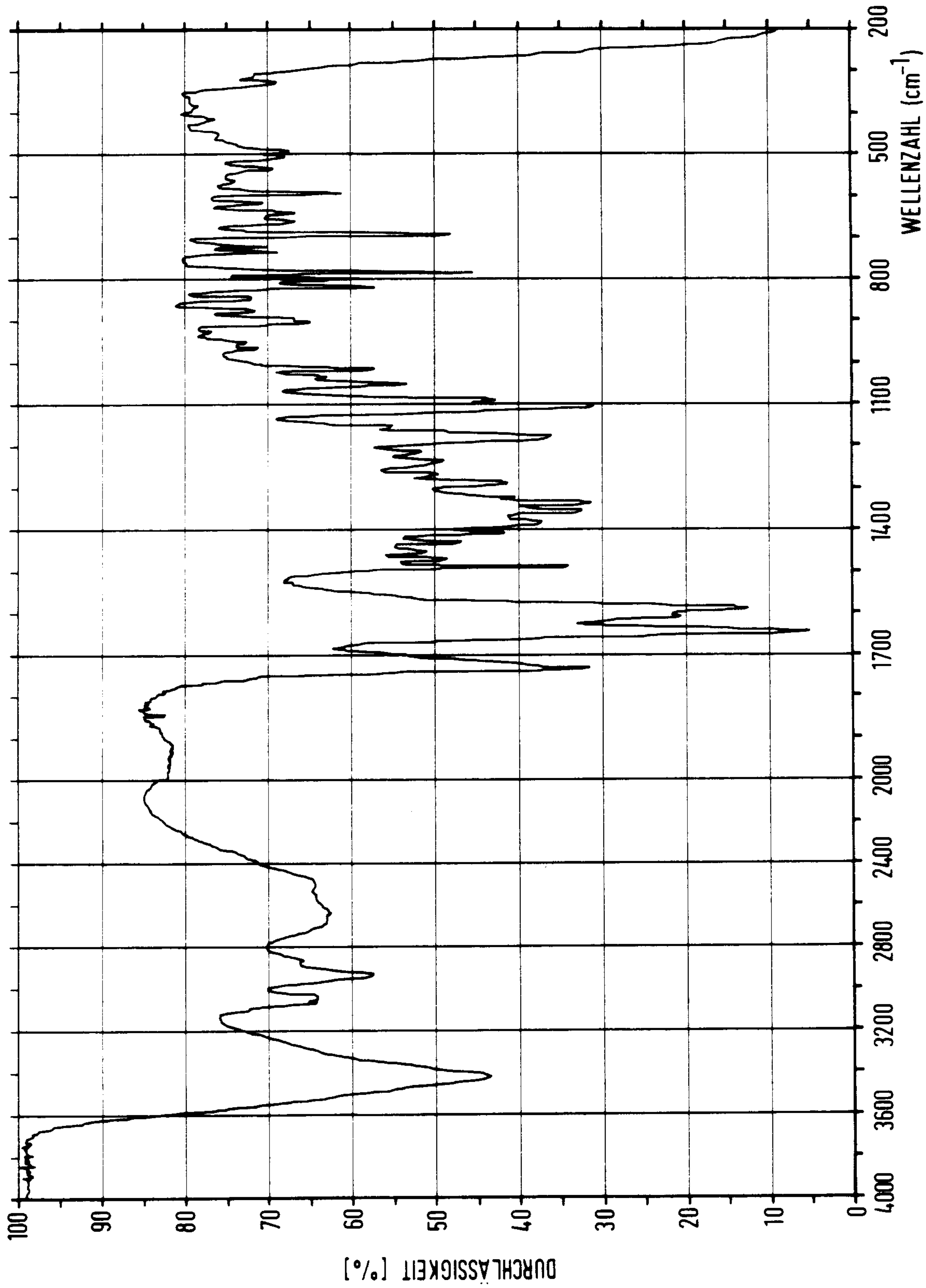
WELLENZAHL (cm-1)
DURCHLÄSSIGKEIT [%]
200
500
800
1100
1400
1700
2000
2400
2800
3200
3600
4000
100
90
80
70
60
50
40
30
20
10
0

Fig. 3

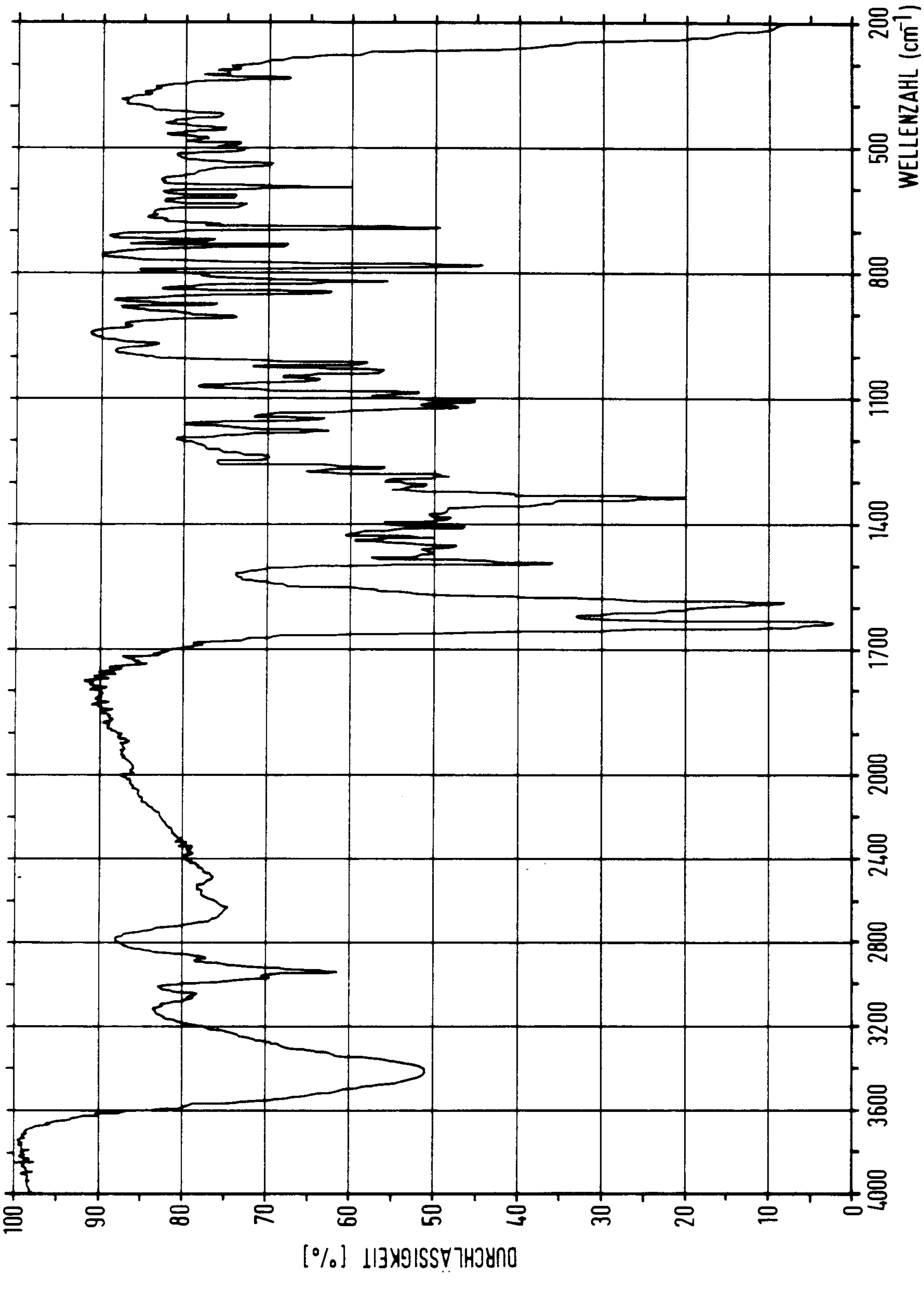
DURCHLÄSSIGKEIT [%]
100
90
80
70
60
50
40
30
20
10
0
WELLENZAHL (cm$^{-1}$)
4000
3600
3200
2800
2400
2000
1700
1400
1100
800
500
200

Fig. 4

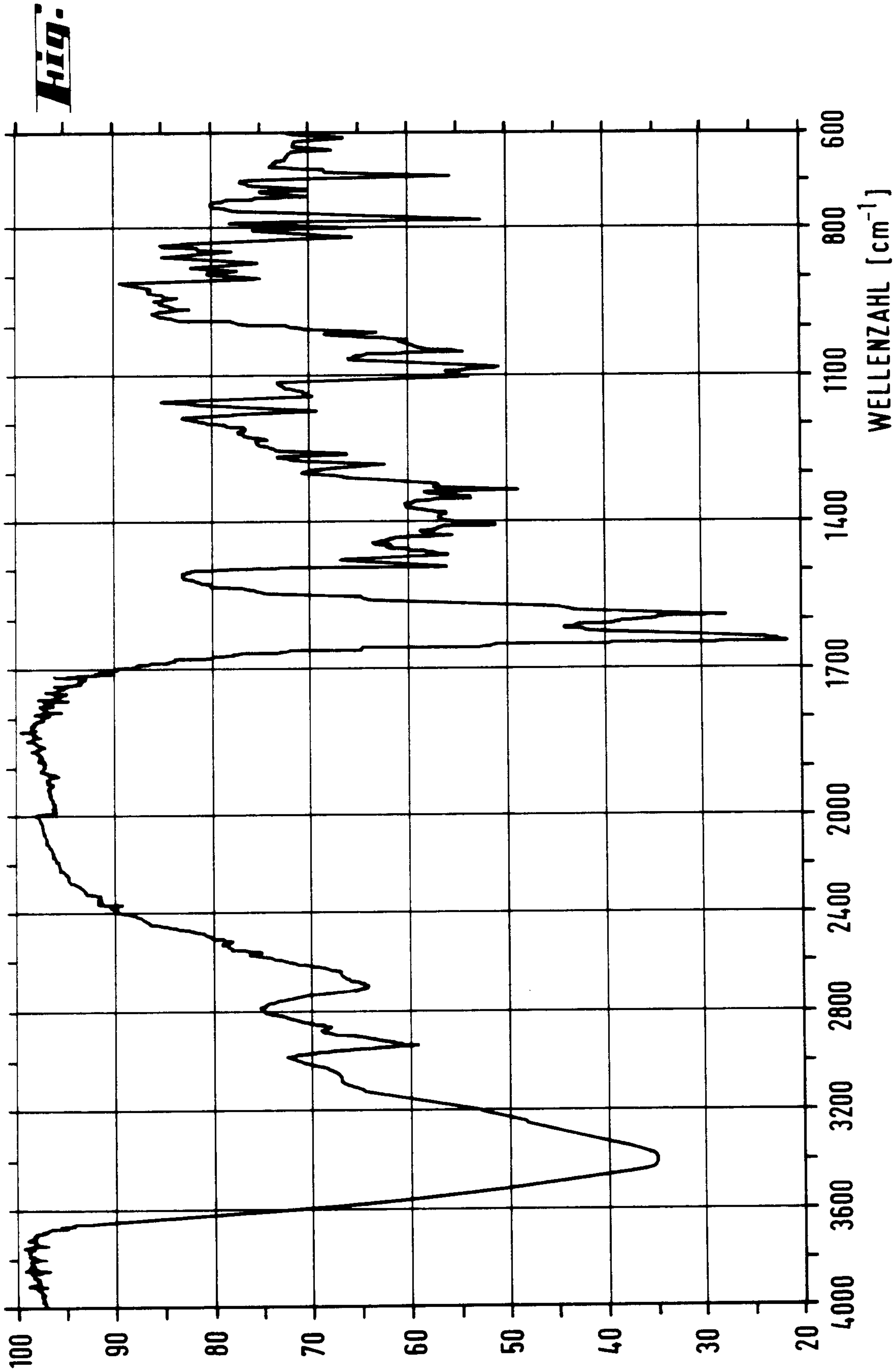
100
90
80
70
60
50
40
30
20
DURCHLÄSSIGKEIT [%]
4000
3600
3200
2800
2400
2000
1700
1400
1100
800
600
WELLENZAHL [$cm^{-1}$]